# EUROPEAN PATENT APPLICATION

(11) **EP 3 243 899 A1**
(43) Date of publication of application: **15.11.2017**
(21) Application number: 15876416.7
(22) Date of filing: 06.01.2015
(51) Int. Cl.: C12M 1/00, C12M 1/26, C12M 1/28, C12M 1/32, G01N 1/00, G01N 1/02

(54) **APPARATUS FOR INSTALLING A SYSTEM SUCH AS A LAB-ON-A-CHIP FOR IDENTIFYING ANTIBIOTIC SUSCEPTIBILITY AT THE POINT OF CARE OF THE PATIENTS**

(71) Applicant: Droguett Bizet, Sara, Santiago 7550188 (CL); Soto Aguilera, Mario, Santiago 7500755 (CL)
(72) Inventor: Droguett Bizet, Sara, Santiago 7550188 (CL); Soto Aguilera, Mario, Santiago 7500755 (CL)
(74) Representative: Giavarini, Francesco
(86) International application number: PCT/CL2015/050002
(87) International publication number: WO 2016/109905

(57) **Abstract**

A lab-on-chip type system, capable of identifying antibiotic sensitivity at the point of care of patients, especially in rural areas, clinics, hospitals that do not have care 24/7, hospitals with low level of equipment, among others, from the extraction of a sample, comprising a micro device or medical base device and a dispenser, wherein said micro device comprises a plurality of microwells, arranged in a circular fashion on one of the faces of the micro device, and wherein the micro-dispenser comprises a central plunger for taking and supplying the sample, a chamber for storage and distribution of the sample and a plurality of microdispensers arranged in a circular manner through which the sample is introduced into the microwells of the micro device.

## Description

### FIELD OF APPLICATION

The present invention relates to the clinical diagnostics industry, in particular to the use of microfluidic devices to perform such diagnoses, such as blood gas analysis, molecular biology-based assays, use of immunoassay at the point of patient care, and other similar analyzes

### BACKGROUND

Different types of microfluidic devices are now known. Labs on a chip or "lab-on-chips" consist of self-sufficient diagnostic platforms in which, in principle, all diagnostic actions can be carried out by mixing between the different reagents, which run through the device and react in suitable cameras, to provide simple readings, generally by visual inspection, about the diagnosis. However, the concept of lab-on-chip, in most situations today corresponds to lab-on-chips devices, that is, a simple microsystem, but it needs a set of machines, pumps, readers, pre-existing support structures in the analysis laboratory, for their correct operation. Current trends are aimed at achieving greater autonomy of these micro fluidic devices so that they can be used in a simple way in the point of attention to the patient. US2009297403 discloses a lab-on-chip or bioreactor system, in addition to the method for making it. The system is complex and has ceramic components, however, unlike other documents, this lab-on-chip system would allow cell growth analysis under defined conditions or could be used as a microbiological reactor.

Another type of micro-devices are the so-called microsystems for total analysis or " -TAS" ("micro total analysis system"), which can be conceived as advanced lab-on-chips or with a greater integration of functions thanks to the use of thermo-opto-electro-mechanical components with which a greater number of physical-chemical domains are controlled and more precise or more detailed answers are obtained.

The prior art also describes the so-called diagnostic strips, which consist of microfluidic devices for diagnosis at the most widespread and simplest point of care, generally oriented to "all / none" diagnoses of the "patient has an infection" type, "Pregnancy tests" and the like. They are usually made of paper or very economical materials (polymers) and act by immersing one of its ends in the sample under study, which diffusion or capillarity reaches different areas of the test strip and activates a color change, In case of positive diagnosis. Recently we are investigating in the passage of the traditional qualitative diagnosis using diagnostic strips to diagnoses with some quantification.

Another type of micro-device described by the prior art corresponds to the so-called fluidic cartridges and integrated platforms, which correspond to complex systems composed of a hardware (desktop machine) with own software to control the analysis process in which cartridges are introduced (As if it were a printer ink cartridge) and with the sample itself to be processed. They have communication ports for their integration into hospital information systems and are often used for complex diagnoses, generally in the fields of genetics, molecular biology and the like.

### TECHNICAL PROBLEM

There is a need for a system that includes a device to identify antibiotic sensitivity at the point of care of patients, especially in rural areas, clinics, hospitals that do not have 24/7 care, hospitals with low level of equipment, among others.

### TECHNICAL SOLUTION

To solve said problem, there is provided a system comprising a circular type micro device, preferably made of medical grade polymer, cyclic olefin or other material which has a plurality of micro-wells for depositing the sample to be treated, and the system further comprises a dispenser for dispensing the sample into each micro-well of the device.

In each microwell the process of identification of the antibiotic sensitivity is developed. Each of the microwells contains an antibiotic, a culture medium and a chromophore or fluorophore substance which would allow the results to be obtained during the day. In the case of urinary infections, the antibiotics to be used would be those routinely used in medical practice, in which Ampicillin, Amoxicillin with Clavulanic Acid, Fosfomycin, Cotrimoxazole, Ciprofloxaxin, Pipedimic Acid, Cefalotoxin, Gentamicin, Nitrofurantoin , Colistin and Vancomycin, which does not exclude the use of other antibiotics.

Each microwell is independent of the others to avoid cross-contamination. In addition, the dispenser comprises a plurality of distribution channels, which coincide in quantity and in position with the microwells of the base, to dispense the sample homogeneously.

### TECHNICAL ADVANTAGES

The technical advantages of the system with the micro device and the dispenser of the invention is that, unlike the state-of-the-art micro devices, its structure allows the identification of antibiotic sensitivity at the point of care of patients in zones, regions, medical facilities without care 24/7, or other types of facilities where there is a low level of equipment. Another advantage is that it allows the ability to identify antibiotic sensitivity at the point of care of the patient without the need to refer the sample to centralized laboratories.

In addition, the system with micro device and dispenser is applicable to the performance of dilution antibiogram according to the client's requirements.

The system is adaptable to the epidemiological conditions of each country or region

It is a ready-to-use product which makes it easy to use in rural areas or where there is no permanent staff or medical equipment.

Accurate and reliable results are obtained more quickly than with the use of the conventional antibiogram.

### BRIEF DESCRIPTION OF THE DRAWINGS

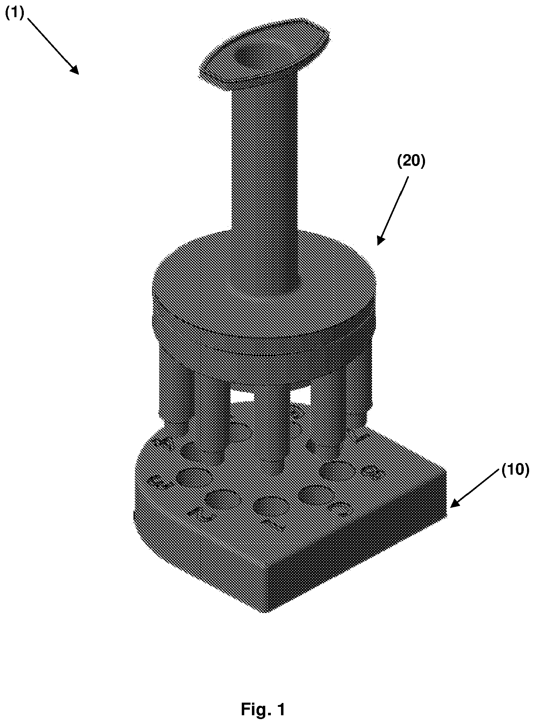

Figure 1 is a view of a lab-on-chip type system according to a preferred embodiment of the invention.

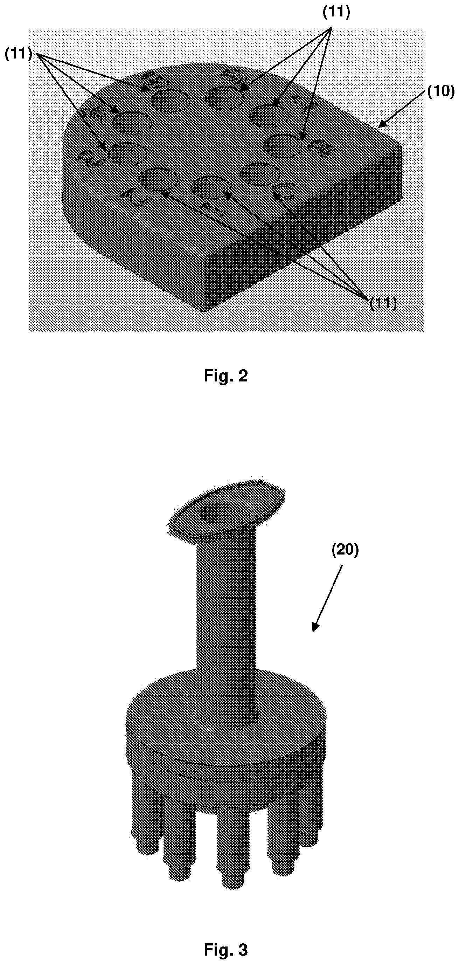

Figure 2 is a view of a base of the system of Figure 1, according to a preferred embodiment of the invention.
Figure 3 is a view of a dispenser of Figure 1, according to a preferred embodiment of the invention.

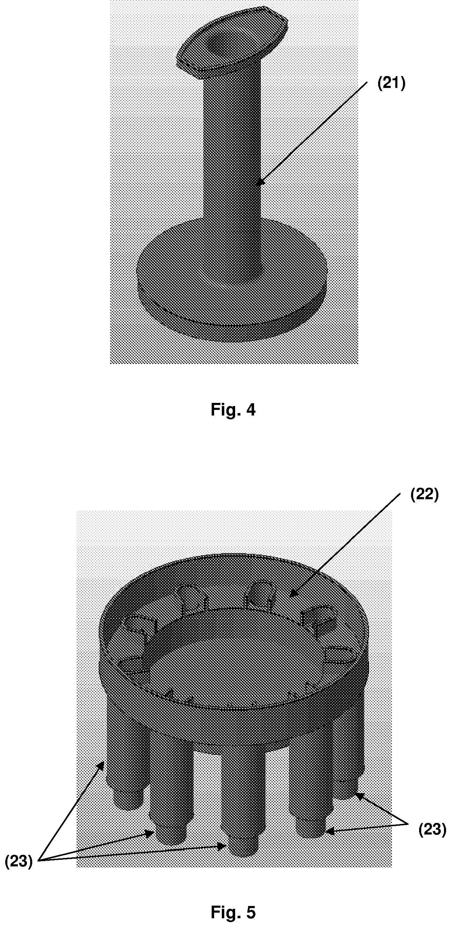

Figure 4 is a view of the upper portion of the dispenser with a plunger according to a preferred embodiment of the invention.
Figure 5 is a view of the lower portion of the dispenser with a storage chamber and a plurality of microdispensers according to a preferred embodiment of the invention.

### DETAILED DESCRIPTION OF THE INVENTION

The invention describes a lab-on-chip type system (1) comprising a micro device or base medical device (10), capable of identifying antibiotic sensitivity at the point of care of patients, especially in rural areas, clinics, hospitals which do not have 24/7 care, hospitals with low level of equipment, transatlantic, among others. The system (1) further comprises a dispenser (20), which is adapted to dispense a sample into the micro device.

The base micro-devices (10) are preferably used in patients with urinary tract infections and the sample preferably to be used is urine.

The base device (10) preferably has some circular portion. Said device (10) is preferably manufactured from a medical grade polymer, cyclic olefin or other material.

The base micro-base (10) comprises a plurality of microwells (11), of preferably circular, in which the process of identifying the antibiotic sensitivity is developed. The microwells (11) are disposed circularly on one of the faces of the micro-device (10).

Each of the microwells (11) of each base device (10) contains an antibiotic, a culture medium and a chromophore or fluorophore substance.

In the case of urinary infections, the antibiotics to be used would be those routinely used in medical practice, in which Ampicillin, Amoxicillin with clavulanic acid, Fosfomycin, Cotrimoxazole, Ciprofloxaxine, Pipedimic acid, Cefalotoxin, Gentamicin, Nitrofurantoin, Colistin and Vancomycin, which does not exclude the use of other antibiotics.

According to the preferred embodiment of the invention, each microwell (11) is independent of the others to avoid cross-contamination.

According to a preferred embodiment of the invention, the quantity of microwells (11) of the base micro-device (10) is at least nine.

In a preferred embodiment of the invention, the dispenser (20) of the system (1) comprises a central plunger (21), a chamber for storage and dispensing of the urine sample (22) preferably with a circular geometry and a plurality of micro- dispensers (23) arranged circularly below the storage chamber (22) through which the sample is introduced into the microwells (11) of the micro device (10). The quantity of micro dispensers (23) coincides with the quantity of microwells (11). According to a preferred embodiment of the invention, the amount of microdispensers (23) of the dispenser (10) are at least nine.

Depending on the incorporation of a chromogenic substance or a fluorophore in the mixture of substances involved in the microbiological process, the time for the detection of antibiotic sensitivity varies from 18 to 20 hours when the indicator is a chromophore, and from 4 to 8 hours when the indicator is a fluorophore.

A number of manufacturing processes can be used to manufacture said micro device (10) and dispenser (11). In these processes, the use of 2D½ and 3D designs, as well as manufacturing technologies by UV photolithography and chemical etching, by laser stereolithography and by casting in silicone molds, stands out because, depending on the level of detail required, the required productivity or of the materials of interest, one can resort to the use of one or the other.

For dispensing the sample, the dispenser (20) is first taken over the sample taken to the patient and the plunger (21) is raised so that the sample enters the microdispensers (23). When the sample is in the microdispensers (23), the dispenser (20) is placed on the base micro-device (10), each micro-dispenser (23) matching its respective micro-well (11). Once this coincidence is achieved, the plunger (21) is lowered to dispense the samples from each micro dispenser (23) to the respective micro-well (11).

## Claims

1. A lab-on-chip type system, capable of identifying antibiotic sensitivity at the point of care of patients, especially in rural areas, clinics, hospitals that do not have care 24/7, hospitals with low level of equipment, among others, **characterized in that** said system comprises a micro device or base medical device and a dispenser comprising a plurality of microwells, arranged in a circular form on one of the faces of the micro device, and wherein the dispenser comprises a central plunger for taking and delivering the sample, a chamber for storing and dispensing the sample and a plurality of microdispensers arranged in a circular shape through which the sample is introduced into the microwells of the micro-device.

2. The system according to any one of claim 1 **characterized in that** it is preferably manufactured from medical grade polymer, cyclic olefin or the like

3. The system according to any one of claim 1 **characterized in that** the microwells are circular.

4. The system according to any one of claim 1 **characterized in that** the microwells are at least nine.

5. The system according to any one of claim 1 **characterized in that** the quantity of micro dispensers (23) coincides with the quantity of microwells (11).

6. The system according to any one of claim 4 **characterized in that** the micro dispensers are at least nine.
